# EUROPÄISCHE PATENTSCHRIFT

(11) **EP 0 796 318 B1**
(45) Veröffentlichungstag und Bekanntmachung des Hinweises auf die Patenterteilung: **31.03.1999**
(21) Anmeldenummer: 95941037.4
(22) Anmeldetag: 29.11.1995
(51) Int. Cl.: C11D 11/00, B01J 2/16, C11D 3/382, C11D 3/12

(54) **FESTE, RIESELFÄHIGE ZUBEREITUNGEN**
SOLID POURABLE PREPARATIONS
PREPARATIONS SOLIDES COULANTES

(30) Priorität: 08.12.1994 DE 4443644
(43) Veröffentlichungstag der Anmeldung: 24.09.1997
(73) Patentinhaber: Henkel Kommanditgesellschaft auf Aktien, 40191 Düsseldorf (DE)
(72) Erfinder: KISCHKEL, Ditmar, D-40789 Monheim (DE); ASSMANN, Georg, D-41363 Jüchen (DE); BECKER, Anke, D-45359 Essen (DE); SCHMID, Karl-Heinz, D-40822 Mettmann (DE); SYLDATH, Andreas, D-40589 Düsseldorf (DE)
(86) Internationale Anmeldenummer: EP9504682
(87) Internationale Veröffentlichungsnummer: WO9617922

(56) Entgegenhaltungen:
- EP-A- 0 191 396
- WO-A-92/01036
- WO-A-92/09265
- DE-A- 2 843 517
- DE-A- 4 127 323
- DE-A- 4 304 062
- US-A- 5 286 400

## Beschreibung

### Gebiet der Erfindung

Die Erfindung betrifft feste, rieselfähige Zubreitungen, die man durch Wirbelschichtgranulierung von wäßrigen Tensidpasten in Gegenwart von Reibkörpern erhält, ein Verfahren zu ihrer Herstellung sowie ihre Verwendung zur Herstellung von Körperreinigungsmitteln.

### Stand der Technik

Zur Reinigung insbesondere von ölverschmutzten Körperteilen haben sich in der Vergangenheit Handwaschpasten als sehr nützlich erwiesen, die neben Tensiden, Abrassivstoffe und Lösungsmittel enthalten. Ein Problem beim Einsatz dieser Pasten besteht jedoch darin, daß sie nur innerhalb eines vergleichsweise engen Temperaturbereiches anwendbar sind und beispielsweise bei höheren Temperaturen zur Phasentrennung neigen, während sie bei niedrigeren Temperaturen erstarren. Außerdem zeigen sie wegen des vergleichsweise geringen Tensidgehaltes eine erhebliche Tendenz zur Verkeimung.

In der Vergangenheit hat man daher versucht, anstelle von Pasten oder Gelen feste, pulverförmige Anbietungsformen zur Verfügung zu stellen, die zusammen mit Wasser auf die Hautoberfläche aufgetragen werden. Hierbei tritt jedoch das Problem auf, daß sich die hautmilden Tenside, die für derartige Formulierungen als tensidische Einsatzstoffe in Betracht kommen wie beispielsweise Sulfosuccinate, Fettalkoholethersulfate oder Alkylglucoside nicht ohne weiteres in wasserfreie Formen überführen lassen. Nach klassischen Verfahren getrocknete wäßrige Pasten dieser Tenside verklumpen leicht und haben die Tendenz unter Aufnahme von Wasser wieder in pastöse Formen überzugehen.

Aus der DE-A-41 27 323 ist ein Verfahren zur Herstellung schwerer, rieselfähiger Tensidgranulate bekannt, wobei flüssige bis pastöse Tensid-Zubereitungsformen gleichzeitig granuliert und getrocknet werden, vorzugsweise in einer kontinuierlich arbeitenden Wirbelschicht. Hinweise auf Tensidgranulate mit Reibkörpern sind der Druckschrift nicht zu entnehmen.

Die Aufgabe der Erfindung hat daher darin bestanden, neue feste, rieselfähige Zubereitungen mit einem Gehalt an Tensiden, insbesondere Sulfosuccinaten, Fettalkoholethersulfaten und/oder Alkylglucosiden einerseits und Reibkörpern andererseits zur Verfügung zu stellen, die frei von den geschilderten Nachteilen sind und die sich für die Herstellung besonders hautverträglicher Körperreinigungsmittel eignen.

### Beschreibung der Erfindung

Gegenstand der Erfindung sind feste, rieselfähige Zubereitungen, die man erhält, indem man wäßrige Tensidpasten zusammen mit Reibkörpern in der Wirbelschicht trocknet und gleichzeitig granuliert.

Überraschenderweise wurde gefunden, daß die gemeinsame Trocknung von wäßrigen Tensidpasten und Reibkörpern in der Wirbelschicht zu Granulaten mit einem schalenförmigen Aufbau führt, die den "Wirkstoff" - also das Tensid - nicht schlagartig, sondern dosiert freisetzen. Hierdurch wird der bei üblichen Produkten bekannte Effekt, daß nach längerem Einwirken der Paste nur noch der Reibkörper auf der Hautoberfläche verbleibt, zuverlässig vermieden. Da die Wirbelschichtgranulierung zu einer zusätzlichen Verrundung der Reibkörper führt, wird bei Anwendung der erfindungsgemäßen Zubereitungen ein verbessertes Glättegefühl erreicht.

Ein weiterer Gegenstand der Erfindung betrifft ein Verfahren zur Herstellung fester, rieselfähiger Zubereitungen, bei dem man wäßrige Tensidpasten zusammen mit Reibkörpern in der Wirbelschicht trocknet und gleichzeitig granuliert.

### Tensidpasten

Grundsätzlich kann das erfindungsgemäße Verfahren auf praktisch alle wäßrigen Tensidsysteme angewendet werden. Bevorzugt sind jedoch wäßrige Pasten derjenigen Systeme, die sich auf anderem Wege nicht oder nur mit hohem technischen Aufwand in eine feste, rieselfähige Form bringen lassen und zudem gute anwendungstechnische Eigenschaften aufweisen. Aus diesem Grunde sind wäßrige Pasten von Fettalkoholsulfaten, Fettalkoholethersulfaten, Sulfosuccinaten, Fettalkoholpolyglycolethern, Alkyl- und/oder Alkenyloligoglykosiden, Fettsäure-N-alkylpolyhydroxyalkylamiden und/oder Betainen bevorzugt. Die Pasten können dabei einen Feststoffgehalt von 25 bis 70 und vorzugsweise 40 bis 60 Gew.-% aufweisen.

### Fettalkoholsulfate

Unter Fettalkoholsulfaten sind die Sulfatierungsprodukte primärer Alkohole zu verstehen, die der Formel (I) folgen,

R¹O-SO₃X (I)

in der R¹ für einen linearen oder verzweigten, aliphatischen Alkyl- und/oder Alkenylrest mit 6 bis 22, vorzugsweise 12 bis 18 Kohlenstoffatomen und X für ein Alkyl- und/oder Erdalkalimetall, Ammonium, Alkylammonium, Alkanolammonium oder Glucammonium steht.

Typische Beispiele für Fettalkoholsulfate, die Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen' schen Oxosynthese erhalten werden. Die Sulfatierungsprodukte können vorzugsweise in Form ihrer Alkalisalze, und insbesondere ihrer Natriumsalze eingesetzt werden. Besonders bevorzugt sind Alkylsulfate auf Basis von C_{16/18}-Talgfettalkoholen bzw. pflanzlicher Fettalkohole vergleichbarer C-Kettenverteilung in Form ihrer Natriumsalze.

### Fettalkoholethersulfate

Fettalkoholethersulfate ("Ethersulfate") stellen bekannte anionische Tenside dar, die großtechnisch durch SO₃- oder CSA-Sulfatierung von Fettalkoholpolyglycolethern und nachfolgende Neutralisation hergestellt werden. Im Sinne der Erfindung kommen Ethersulfate in Betracht, die der Formel **(II)** folgen,

R²O-(CH₂CH₂O)ₘSO₃X (II)

in der R¹ für einen linearen oder verzweigten Alkyl- und/oder Alkenylrest mit 6 bis 22 Kohlenstoffatomen, n für Zahlen von 1 bis 10 und X für ein Alkali- und/oder Erdalkalimetall, Ammonium, Alkylammonium, Alkanolammonium oder Glucammonium steht.

Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technische Mischungen, in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von im Mittel 2 bis 3 Mol Ethylenoxid an technische C_{12/14}- bzw. C_{12/18}-Kokosfettalkoholfraktionen in Form ihrer Natrium- und/ oder Magnesiumsalze.

### Sulfosuccinate

Sulfosuccinate, die auch als Sulfobernsteinsäureester bezeichnet werden, stellen bekannte anionische Tenside dar, die nach den einschlägigen Methoden der präparativen organischen Chemie erhalten werden können. Sie folgen der Formel **(III)**, 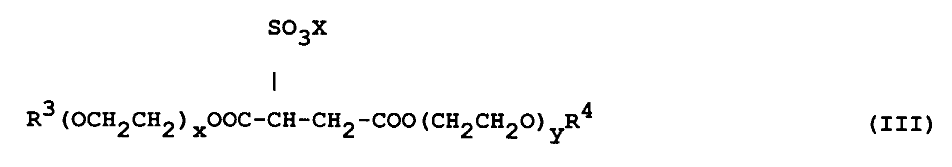 in der R³ für einen Alkyl- und/oder Alkenylrest mit 6 bis 22 Kohlenstoffatomen, R⁴ für R³ oder X, x und y unabhängig voneinander für 0 oder Zahlen von 1 bis 10 und X für ein Alkali- oder Erdalkalimetall, Ammonium, Alkylammonium, Alkanolamonium oder Glucamonium steht.

Zu ihrer Herstellung geht man üblicherweise von Maleinsäure, vorzugsweise aber Maleinsäureanhydrid aus, die im ersten Schritt mit gegebenenfalls ethoxylierten primären Alkoholen verestert werden. An dieser Stelle kann durch Variation von Alkoholmenge und Temperatur das Mono-/Diester-Verhältnis eingestellt werden. Im zweiten Schritt erfolgt die Anlagerung von Bisulfit, die üblicherweise im Lösungsmittel Methanol durchgeführt wird. Neuere Übersichten zu Herstellung und Verwendung von Sulfosuccinaten sind beispielsweise von T.Schoenberg in **Cosm.Toil. 104, 105 (1989),** J.A.Milne in **R.Soc.Chem. (Ind.Appl.Surf.II) 77, 77 (1990)** sowie W.Hreczuch et al. in **J.Am.Oil.Chem.Soc. 70, 707 (1993)** erschienen.

Typische Beispiele sind Sulfobernsteinsäuremono- und/oder -diester in Form ihrer Natriumsalze, die sich von Fettalkoholen mit 8 bis 18, vorzugsweise 8 bis 10 bzw. 12 bis 14 Kohlenstoffatomen ableiten; die Fettalkohole können dabei mit durchschnittlich 1 bis 10 und vorzugsweise 1 bis 5 Mol Ethylenoxid verethert sein und dabei sowohl eine konventionelle als auch vorzugsweise eine eingeengte Homologenveteilung aufweisen. Exemplarisch genannt seien Di-n-octylsulfosuccinat und Monolauryl-3EO-sulfosuccinat in Form ihrer Natriumsalze.

### Fettalkoholpolyglycolether

Fettalkoholpolyglycolether stellen bekannte nichtionische Tenside dar, die großtechnisch durch Alkoxylierung von Fettalkoholen in Gegenwart von sauren, vorzugsweise jedoch alkalischen Katalysatoren hergestellt werden. Dabei kann es sich bei den Polyglycolethern um Ethylenoxid (EO)-, Propylenoxid (PO)- oder EO/PO-Addukte - letztere mit Random- oder Blockverteilung - handeln. Im Sinne der Erfindung kommen Fettalkoholpolyglycolether der Formel (IV) in Betracht,

R⁵O-(CH₂CH₂O)_{z}H (IV)

in der R⁵ für einen linearen oder verzweigten Alkyl- und/oder Alkenylrest mit 6 bis 22 Kohlenstoffatomen und z für Zahlen von 1 bis 10 steht.

Typische Beispiele sind Anlagerungsprodukte von durchschnittlich 1 bis 10 und insbesondere 3 bis 7 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technische Mischungen, in Form ihrer Natrium- und/oder Magnesiumsalze. Die Polyglycolether können dabei sowohl eine konventionelle als auch eine eingeengte Homologenverteilung aufweisen. Besonders bevorzugt ist der Einsatz von Addukten von durchschnittlich 3 bis 7 Mol Ethylenoxid an technische Kokosfettalkohol- bzw. Oleylalkoholfraktionen

### Alkyl- und/oder Alkenyloligoglucoside

Alkyl- und Alkenyloligoglykoside stellen bekannte Stoffe dar, die nach den einschlägigen Verfahren der präparativen organischen Chemie erhalten werden können und der Formel **(V)** folgen,

R⁶O-[G]ₚ (V)

in der R⁶ für einen Alkyl- und/oder Alkenylrest mit 4 bis 22 Kohlenstoffatomen, G für einen Zuckerrest mit 5 oder 6 Kohlenstoffatomen und p für eine Zahl von 1 bis 10 steht. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside.**

Die Indexzahl p in der allgemeinen Formel (V) gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R⁶ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R⁶ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide stellen ebenfalls bekannte nichtionische Tenside dar und folgen der Formel (VI), 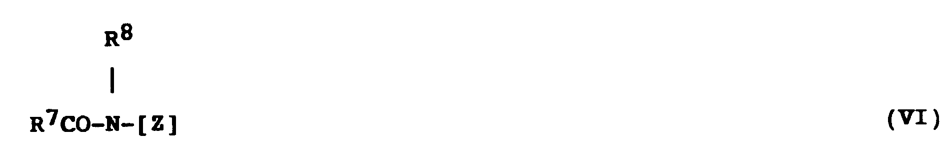 in der R⁷CO für einen aliphatischen Acylrest mit 6 bis 22 Kohlenstoffatomen, R⁸ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1985424, US 2016962** und **US 2703798** sowie die Internationale Patentanmeldung **WO 92/06984** verwiesen. Eine Übersicht zu diesem Thema von H.Kelkenberg findet sich in **Tens. Surf.Det. 25, 8 (1988)**.

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(VII)** wiedergegeben werden: 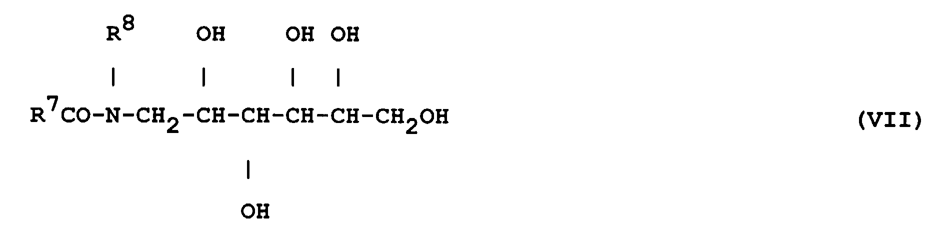 Vorzugsweise werden als Fettsäure-N-alkylpolyhydroxyalkylamide Glucamide der Formel **(VII)** eingesetzt, in der R⁸ für Wasserstoff, einen Alkyl- oder Hydroxyalkylrest mit 1 bis 4 Kohlenstoffatomen steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel **(VII)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Betaine

Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren wie beispielweise Acrylsäure möglich. Zur Nomenklatur und insbesondere zur Unterscheidung zwischen Betainen und "echten" Amphotensiden sei auf den Beitrag von U.Ploog in **Seifen-Öle- Fette-Wachse, 198, 373 (1982)** verwiesen. Weitere Übersichten zu diesem Thema finden sich beispielsweise von A.O'Lennick et al. in **HAPPI, Nov. 70 (1986),** S.Holzman et al. in **Tens.Det. 23, 309 (1986)**, R.Bibo et al. in **Soap Cosm.Chem.Spec. Apr. 46 (1990)** und P.Ellis et al. in **Euro Cosm. 1, 14 (1994)**.

Beispiele für geeignete Betaine stellen die Carboxyalkylierungsprodukte von sekundären und insbesondere tertiären Aminen dar, die der Formel **(VIII)** folgen, 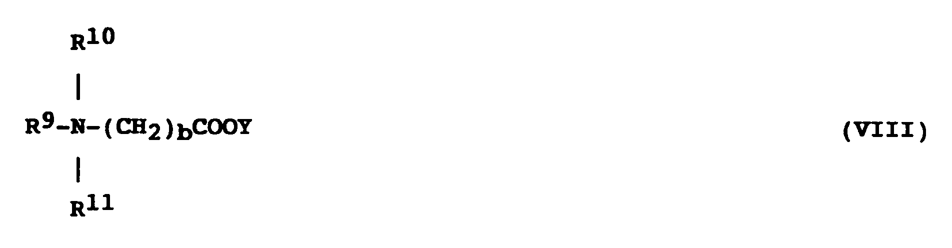 in der R⁹ für Alkyl- und/oder Alkenylreste mit 6 bis 22 Kohlenstoffatomen, R¹⁰ für Wasserstoff oder Alkylreste mit 1 bis 4 Kohlenstoffatomen, R¹¹ für Alkylreste mit 1 bis 4 Kohlenstoffatomen, b für Zahlen von 1 bis 6 und Y für ein Alkaliund/oder Erdalkalimetall oder Ammonium steht.

Typische Beispiele sind sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von **Amidoaminen** in Betracht, die der Formel **(IX)** folgen, 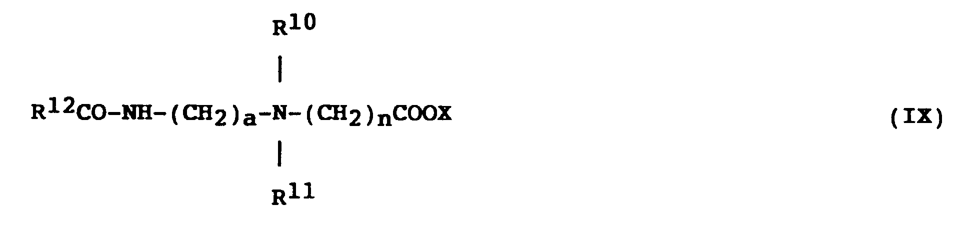 in der R¹²CO für einen aliphatischen Acylrest mit 6 bis 22 Kohlenstoffatomen und 0 oder 1 bis 3 Doppelbindungen, a für Zahlen von 1 bis 3 steht und R¹⁰, R¹¹, b und Y die oben angegebenen Bedeutungen haben.

Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat.

Weiterhin kommen als geeignete Ausgangsstoffe für die im Sinne der Erfindung einzusetzenden Betaine auch **Imidazoline** in Betracht, die der Formel **(X)** folgen, 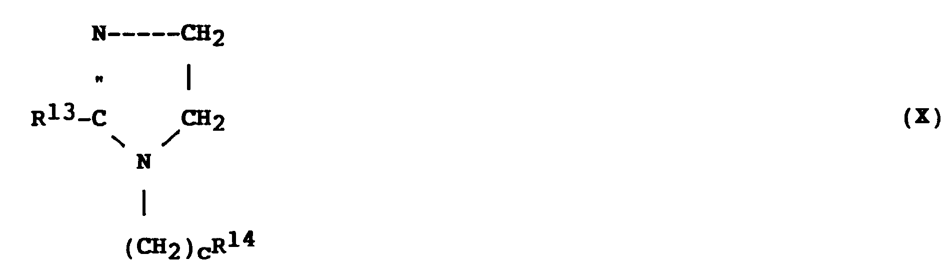 in der R¹³ für einen Alkylrest mit 5 bis 21 Kohlenstoffatomen, R¹⁴ für eine Hydroxylgruppe, einen OCOR¹³- oder NHCOR¹³-Rest und c für 2 oder 3 steht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar.

Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

### Reibkörper

Als geeignete Reiberkörper kommen beispielsweise Holzmehl, geriebener Bimstein, Nußschalen- und/oder Pflanzenfasermahlgut in Betracht. Typische Beispiele sind gemahlene Holzfaserstoffe (Lignocell^{(R)} BK 40/90, lichte Maschenweite 150 µm : max. 95 %), Walnußschalengranulat (UNG 300, Rettenmaier & Co., Ellwangen/FRG, Partikelbereich 170 bis 230 µm) oder geschrotete Rückstände aus der Maisgewinnung. Es können auch gemahlene Kunststoffe eingesetzt werden, sofern diese unter den Bedingungen der Wirbelschicht nicht schmelzen. Die Tenside - bezogen auf Feststoffgehalt - und die Reibkörper können im Gewichtsverhältnis 60 : 40 bis 5 : 95, vorzugsweise 50 : 50 bis 10 : 90 eingesetzt werden.

Insbesondere bei Einsatz größerer Mengen Reibkörper können diese durch die Zugabe von anorganischen und oder organischen Salzen als Co-Trägern partiell substituiert werden. Hierfür kommen Salze wie beispielsweise Alkali- und/oder Erdalkalisulfate, -Chloride, -Hydrogencarbonate, neutrale Silicate sowie Citrate in Betracht, solange die unter ihrer Mitverwendung hergestellten Zubereitungen auf der Haut einen pH-Wert im Bereich von 6 bis 9, vorzugsweise 7 bis 8 aufweisen.

### Zusatzstoffe

Die erfindungsgemäßen Zubereitungen können als Zusatzstoffe beispielsweise Emulgatoren und/oder Schaumbooster enthalten. Typische Beispiele für geeignete O/W- bzw. W/O-Emulgatoren sind gehärtetes und ethoxyliertes Ricinusöl, Polyglycerinfettsäureester oder Polyglycerinpolyricinoleate. Als Schaumbooster eignen sich z.B. Fettsäurealkanolamide. Sofern die Zusatzstoffe unter den Bedingungen der Wirbelschicht thermisch stabil sind, können sie dem Gut bereits vor der Granulierung/Trocknung zugegeben werden. Falls dies nicht der Fall ist, wie beispielsweise bei Farb- und Duftstoffen, können sie auch nachträglich (z.B. durch Versprühen) auf das Gut gebracht werden. Der Anteil der Zusatzstoffe kann 1 bis 10, vorugsweise 2 bis 5 Gew.-% - bezogen auf das resultierende Granulat - betragen.

### Wirbelschichtgranulierung

Unter der Wirbelschicht- oder SKET-Granulierung versteht der Fachmann ein kontinuierliches oder diskontinuierliches Verfahren, bei dem man wäßrige Tensidpasten gleichzeitig trocknet und in stückige Form bringt. Der Granulation wird kontinuierlich Reibkörper zugeführt, auf den die wässrige Tensidpaste aufgesprüht wird. Bevorzugt eingesetzte Wirbelschicht-Apparate besitzen Bodenplatten mit Abmessungen von 0,4 bis 5 m. Vorzugsweise wird die SKET-Granulierung bei Wirbelluftgeschwindigkeiten im Bereich von 1 bis 8 m/s durchgeführt. Der Austrag der Granulate aus der Wirbelschicht erfolgt vorzugsweise über eine Größenklassierung der Granulate. Die Klassierung kann beispielsweise mittels einer Siebvorrichtung oder durch einen entgegengeführten Luftstrom (Sichterluft) erfolgen, der so reguliert wird, daß erst Teilchen ab einer bestimmten Teilchengröße aus der Wirbelschicht entfernt und kleinere Teilchen in der Wirbelschicht zurückgehalten werden.

Üblicherweise setzt sich die einströmende Luft aus der beheizten oder unbeheizten Sichterluft und der beheizten Bodenluft zusammen. Die Bodenlufttemperatur liegt dabei zwischen 80 und 180, vorzugsweise 90 und 140°C, wobei es gerade im Hinblick auf die Mitverwendung von Zuckerttensiden empfehlenswert ist, die Temperatur der Masse in der Wirbelschicht auf 70 bis 90°C zu begrenzen. Vorteilhafterweise wird weiterhin zu Beginn der SKET-Granulierung eine Startmasse, beispielsweise der Reibkörper oder ein SKET-Granulat aus einem früheren Versuchsansatz, vorgelegt. In der Wirbelschicht verdampft das Wasser aus den Tensidpasten, wobei angetrocknete bis getrocknete Keime entstehen, die mit weiteren Mengen Tensid umhüllt, granuliert und wiederum gleichzeitig getrocknet werden.

### Gewerbliche Anwendbarkeit

Die festen Zubereitungen weisen einen Wassergehalt von unter 5 und vorzugsweise unter 1 Gew.-% auf, sind sehr gut rieselfähig und gegen Verkeimung stabilisiert. Sie eignen sich zur Herstellung von Körperreinigungsmitteln wie insbesondere Handwaschpulvern. Sie können auch in lösungsmittelhaltige Formulierungen eingearbietet werden und dann zur Herstellung von Handwaschpasten dienen.

Die folgenden Beispiele sollen den Gegenstand der Erfindung näher erläutern.

### Beispiele

### I. Einsatzstoffe

A1) Di-Natrium Laurethsulfosuccinat (Texapon^{(R)} SB3)
A2) Coco Betaine (Dehyton^{(R)} AB 30)
A3) Decyl Polyglucose (Plantaren^{(R)} 2000)
A4) Kokosfettsäure-N-methylglucamid
A5) Natrium Laurethsulfat (and) Magnesium Laurethsulfat (Texapon^{(R)} ASV)
A6) Kokosalkoholsulfat-Natriumsalz (Sulfopon^{(R)} KT 115
A7) Kokosalkohol+3EO-Addukt (Dehydol^{(R)} LT3)
A8) Oleylalkohol+7EO-Addukt (Eumulgin^{(R)} WM7)

B1) Walnußschalengranulat (UNG 300)
B2) Natriumsulfat

C1) Kokosfettsäurediethanolamid (Comperlan^{(R)} KD

Die tensidischen Einsatzstoffe Al bis A8 sowie der Zusatzstoff C1 sind Produkte der Henkel KGaA, Düsseldorf/FRG. Die Stoffe A1 bis A7 und C1 wurden als wäßrige Pasten mit einem Feststoffgehalt von 30 bis 55 Gew.-% eingesetzt, die Stoffe A7 und A8 sind wasserfrei, jedoch flüssig.

Der Reibkörper B1 stellt ein Produkt der Firma J.Rettenmaier, Ellwangen/FRG dar. Die Rezepturen sind in Tabelle 1 zusammengefaßt (Angaben als Gew.-% bezogen auf Feststoff).

**Tabelle 1**

| Rezepturbeispiele | | | | | | | | | | | |
|---|---|---|---|---|---|---|---|---|---|---|---|
| **R** | **A1** | **A2** | **A3** | **A4** | **A5** | **A6** | **A7** | **A8** | **B1** | **B2** | **C1** |
| R1 | 10 | - | - | - | - | - | - | - | 90 | - | - |
| R2 | - | 5 | 5 | - | - | - | - | - | 45 | 45 | - |
| R3 | - | 20 | - | - | 20 | - | - | - | 60 | - | - |
| R4 | 15 | - | 5 | - | - | - | - | - | 60 | 20 | - |
| R5 | 15 | - | - | 5 | - | - | - | - | 70 | 30 | - |
| R6 | 10 | 10 | - | - | - | 10 | - | - | 50 | 20 | - |
| R7 | 10 | - | - | - | - | 30 | - | - | 60 | - | - |
| R8 | 35 | - | - | - | - | - | - | 10 | 40 | - | 5 |
| R9 | 20 | 5 | 5 | - | - | - | 10 | - | 45 | - | 5 |

### II. Herstellung der SKET-Granulate in der Wirbelschicht

Zur Herstellung der Granulate wurde der Reibkörper in der Granulationsanlage vorgelegt und das Tensid bzw. die Tensidpaste aufgesprüht. Während des Prozesses wurde laufen Reibkörper zudosiert, bis hinsichtlich der angestrebten Rezepturen R1 bis R9 ein stationärer Zustand erreicht wurde.

### Kenndaten des Verfahrens:

Wirbelschicht,
- Durchmesser 400 mm
- Fläche 0,13 m²
- Luftgeschwindigkeit 3,8 m/s

Temperatur,
- Bodenluft 132 °C
- Sichterluft 20 °C
- Luftaustritt 73 °C
- Schichtmasse 82 °C

Luftstrom 1300 m³/h
Luftbeladung 11 g H₂O/kg Luft

Durchsatz,
- Tensid 33,3 kg/h
- Reibkörper 90 kg/h
- Startmasse (Reibkörper) 20 kg

Es wurden in allen Fällen staubfreie, rieselfähige Granulate erhalten. Die Siebanalyse für das Produkt entsprechend Rezepturbeispiel R1 lautet:

| | |
|---|---|
| > 1,6 mm | 9,7 Gew.-% |
| 0,8 mm | 45,3 Gew.-% |
| 0,6 mm | 22,1 Gew.-% |
| 0,4 mm | 17,5 Gew.-% |
| 0,2 mm | 4,6 Gew.-% |
| < 0,1 mm | 0,8 Gew.-% |

## Patentansprüche

1. Feste, rieselfähige Zubereitungen, dadurch erhältlich, daß man wäßrige Tensidpasten zusammen mit Reibkörpem, ausgewählt aus der Gruppe, die gebildet wird von geriebenem Bimsstein, Holzmehl, Nußschalen- und/oder Pflanzenfasermahlgut, in der Wirbelschicht trocknet und gleichzeitig granuliert.

2. Verfahren zur Herstellung fester, rieselfähiger Zubereitungen durch Wirbelschichttrocknung und gleichzeitige Granulierung von wäßrigen Tensidpasten, **dadurch gekennzeichnet**, daß man Reibkörper mitverwendet, die ausgewählt sind aus der Gruppe, die gebildet wird von geriebenem Bimsstein, Holzmehl, Nußschalen- und/oder Pflanzenfasermahlgut.

3. Verfahren nach Anspruch 2, **dadurch gekennzeichnet**, daß man Tenside einsetzt, die ausgewählt sind aus der Gruppe, die gebildet wird von Fettalkoholsulfaten, Fettalkoholethersulfaten, Sulfosuccinaten, Fettalkoholpolyglycolethern, Alkyl- und/oder Alkenyloligoglykosiden, Fettsäure-N-alkylpolyhydroxyalkylamiden und/oder Betainen.

4. Verfahren nach den Ansprüchen 2 und 3, **dadurch gekennzeichnet**, daß man wäßrige Tensidpasten mit einem Feststoffgehalt von 25 bis 70 Gew.-% einsetzt.

5. Verfahren nach den Ansprüchen 2 bis 4, **dadurch gekennzeichnet**, daß man die Tenside - bezogen auf Feststoffgehalt - und die Reibkörper im Gewichtsverhältnis 60 : 40 bis 5 : 95 einsetzt.

6. Verfahren nach den Ansprüchen 2 bis 5, **dadurch gekennzeichnet,** daß man als Zusatzstoffe Emulgatoren und/ oder Schaumbooster einsetzt.

7. Verfahren nach Anspruch 6, **dadurch gekennzeichnet**, daß man die Zusatzstoffe in Mengen von 1 bis 10 Gew.-% - bezogen auf das resultierende Granulat - einsetzt.

8. Verfahren nach den Ansprüchen 2 bis 7, **dadurch gekennzeichnet**, daß man die Temperatur der Masse in der Wirbelschicht auf 70 bis 90°C begrenzt.

9. Verwendung von festen, rieselfähigen Zubereitungen nach Anspruch 1 zur Herstellung von Körperreinigungsmitteln.

## Claims

1. Solid free-flowing preparations obtainable by fluidized bed drying and, at the same time, granulation of aqueous surfactant pastes together with abrasive particles selected from the group consisting of ground pumice stone, wood meal, ground nut shells and/or vegetable fibres.

2. A process for the production of solid free-flowing preparations by fluidized bed drying and, at the same time, granulation of aqueous surfactant pastes, characterized in that abrasive particles selected from the group consisting of ground pumice stone, wood meal, ground nut shells and/or vegetable fibres are used.

3. A process as claimed in claim 2, characterized in that surfactants selected from the group consisting of fatty alcohol sulfates, fatty alcohol ether sulfates, sulfosuccinates, fatty alcohol polyglycol ethers, alkyl and/or alkenyl oligoglycosides, fatty acid-N-alkyl polyhydroxyalkyl amides and/or betaines are used.

4. A process as claimed in claims 2 and 3, characterized in that aqueous surfactant pastes with a solids content of 25 to 70% by weight are used.

5. A process as claimed in claims 2 to 4, characterized in that the surfactants - based on solids content - and the abrasive particles are used in a ratio by weight of 60:40 to 5:95.

6. A process as claimed in claims 2 to 5, characterized in that emulsifiers and/or foam boosters are used as additives.

7. A process as claimed in claim 6, characterized in that the additives are used in quantities of 1 to 10% by weight, based on the resulting granules.

8. A process as claimed in claims 2 to 7, characterized in that the temperature of the paste in the fluidized bed is limited to 70-90°C.

9. The use of the solid free-flowing preparations claimed in claim 1 for the production of body-cleansing products.

## Revendications

1. Préparations solides coulantes que l'on peut obtenir en séchant dans un lit fluidisé et simultanément en granulant des pâtes tensioactives aqueuses avec des corps nettoyants choisis dans le groupe formé par la pierre ponce broyée, la farine de bois, les broyats de coquille de noix et/ou de fibres végétales.

2. Procédé de fabrication de préparations solides coulantes par séchage en lit fluidisé et simultanément granulation de pâtes tensioactives aqueuses,
caractérisé en ce qu'
on utilise des corps nettoyants qui sont choisis dans le groupe formé par la pierre ponce broyée, la farine de bois, le broyat de coquille de noix et/ou de fibres végétales.

3. Procédé selon la revendication 2,
caractérisé en ce qu'
on utilise des agents tensioactifs qui sont choisis dans le groupe formé par les sulfates d'alcools gras, les éthers sulfates d'alcools gras, les sulfosuccinates, les polyglycols éthers d'alcools gras, les alkyl- et/ou alcényloligoglycosides, les N-alkyl-polyhydroxyalkylamides d'acides gras et/ou les bétaïnes.

4. Procédé selon les revendications 2 et 3,
caractérisé en ce qu'
on utilise des pâtes tensioactives aqueuses ayant une teneur en solides de 25 à 70 % en poids.

5. Procédé selon les revendications 2 à 4,
caractérisé en ce qu'
on utilise les agents tensioactifs - par rapport à la teneur en solides - et les corps nettoyants dans un rapport pondérai de 60:40 à 5:95.

6. Procédé selon les revendications 2 à 5,
caractérisé en ce qu'
on utilise comme additifs des émulsifiants et/ou des exaltateurs de mousse.

7. Procédé selon la revendication 6,
caractérisé en ce qu'
on utilise les additifs à des quantités de 1 à 10 % en poids par rapport au granulé résultant.

8. Procédé selon les revendications 2 à 7,
caractérisé en ce qu'
on limite la température de la masse dans le lit fluidisé à 70 à 90°C.

9. Utilisation de préparations solides coulantes selon la revendication 1 pour la production d'articles de nettoyage du corps.
